(19) Europäisches Patentamt
European Patent Office
Office européen des brevets

(11) Numéro de publication: **0 030 514**
**B1**

(12) **FASCICULE DE BREVET EUROPEEN**

(45) Date de publication du fascicule du brevet: **17.08.83**

(51) Int. Cl.³: **C 07 C 87/60**, C 07 C 87/62, A 01 N 33/06

(21) Numéro de dépôt: **80420140.8**

(22) Date de dépôt: **10.12.80**

(54) **Dérivés de l'aniline, procédé pour leur préparation et compositions régulatrices de la croissance des plantes les contenant.**

(30) Priorité: **11.12.79 FR 7930803**

(43) Date de publication de la demande:
**17.06.81 Bulletin 81/24**

(45) Mention de la délivrance du brevet:
**17.08.83 Bulletin 83/33**

(84) Etats contractants désignés:
**AT BE CH DE FR GB IT LI LU NL**

(56) Documents cités:
**DE-A-2 343 606**
**FR-A-2 040 427**
**JOURNAL OF THE AMERICAN CHEMICAL SOCIETY, vol. 90, no. 13, 19 juin 1968, R. C. HAHN et al.: »Electrical Effects of cycloalkyl groups«, pages 3404—3415**

(73) Titulaire: **RHONE-POULENC AGROCHIMIE, 14-20, rue Pierre Baizet, F-69009 Lyon (FR)**

(72) Inventeur: **Guigues, François, 936, avenue Victor Hugo, F-69140 Rillieux (FR)**
Inventeur: **Borrod, Guy, 38 bis, rue des Granges, F-69005 Lyon (FR)**

(74) Mandataire: **Chaumette, Michel et al, RHONE-POULENC AGROCHIMIE BP 9163-09, F-69263 Lyon Cedex 1 (FR)**

EP 0 030 514 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Dérivés de l'aniline, procédé pour leur préparation et compositions régulatrices de la croissance des plantes les contenant

La présente invention concerne des compositions régulatrices de la croissance des plantes contenant comme matière active des dérivés de cyclopropylaniline ainsi que les traitements effectués au moyen de ces dérivés. Elle concerne également les nouveaux dérivés de cyclopropylaniline utilisables comme matière active dans les compositions selon l'invention, ainsi que la préparation de ces dérivés.

Dans le texte de la présente demande de brevet, les expressions »régulatrices de la croissance« et »régulateurs de croissance« sont prises dans leur acception courante en langue française, qui correspond à »substances de croissance« de la littérature anglo-saxonne, le mot »croissance« se rapportant à la production de matière vivante et non simplement à la modification de la taille des plantes. Par »régulateurs de croissance«, on entendra donc dans la suite des produits capables de modifier la physiologie des plantes de diverses manières.

Les compositions régulatrices de la croissance des plantes, selon l'invention, sont caractérisées en ce qu'elles contiennent comme matière active au moins un dérivé de cyclopropylaniline répondant à la formule (I):

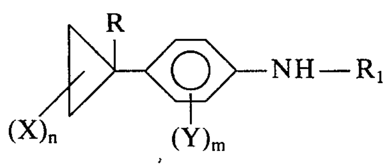

(I)

dans laquelle:

- R représente un atome d'hydrogène ou un radical alcoyle contenant de 1 à 4 atomes de carbone,
- X et Y, identiques ou différents, représentent chacun un atome d'halogène,
- $R_1$ représente un atome d'hydrogène ou un radical alcoyle contenant de 1 à 6 atomes de carbone,
- n est un nombre entier égal à 0, 1 ou 2, les différents atomes X, lorsque n est supérieur à 1, pouvant être identiques ou différents,
- m est un nombre entier égal à 0, 1, 2, 3 ou 4, les différents atomes Y, lorsque m est supérieur à 1, pouvant être identiques ou différents.

Certains dérivés de cyclopropylaniline ont déjà été décrits dans la littérature, ainsi:

- la demande de brevet allemand No DE-A-2 343 606 décrit la (dichloro-2,2 cyclopropyl)-4 aniline en tant que produit de départ pour la préparation de médicaments,
- la revue: Journal of the American Chemical Society, Vol. 90, No 13, 19 juin 1968, pages 3404–3415, décrit la cyclopropyl-4 aniline ainsi que son utilisation pour la préparation du bromo-1 cyclopropyl-4 benzène.

Toutefois, aucun de ces documents ne mentionne, ni même ne suggère la possibilité d'une activité régulatrice de la croissance des plantes pour les composés qui y sont décrits.

Par ailleurs, enfin le FR-A-2 040 427 revendique en tant qu'insecticides et acaricides des composés de formule

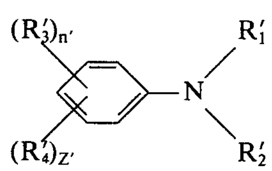

dans laquelle $R'_1$, et $R'_2$ et $R'_3$ sont chacun des atomes d'hydrogène ou des groupes alkyles, alkényles ou alkinyles portant ou non des substituants, $R'_4$ est un atome d'halogène, n' est égal à 1, 2 ou 3 et Z' à 0, 1, 2 ou 3.

Dans cette formule, $R'_3$ représente un radical alkyle ($C_1 - C_{18}$) linéaire du ramifié. Toutefois, ce brevet ne mentionne pas pour $R'_3$ la possibilité d'être un radical cycloalkyle tel que par exemple le cyclopropyle. Enfin, si on se réfère aux nombreux exemples décrits dans ce brevet, on observera que dans tous ces exemples $R'_3$ représente soit un alkyle linéaire, soit un alkyle ramifié, mais jamais un cycloalkyle.

Les composés selon la formule I de notre demande sont donc différents de ceux décrits dans ce FR-A-2 040 427. Leurs propriétés de régulateurs de la croissance des plantes étaient tout à fait inattendues compte tenu de ce document qui décrit des propriétés insecticides et acaricides.

En plus des compositions décrites précédemment, l'invention concerne, à titre de produits nouveaux, les composés répondant à la formule (I), à l'exclusion des deux composés décrits dans la littérature, c'est-à-dire la (dichloro-2,2 cyclopropyl)-4 aniline et la cyclopropyl-4 aniline.

Les composés selon la formule (I) peuvent former des sels par réaction avec un acide approprié (par exemple l'acide chlorhydrique, l'acide sulfurique, l'acide nitrique, l'acide phosphoreux etc . . .). Ces sels ainsi que les compositions les contenant sont compris dans le cadre de l'invention. Parmi ces sels, on peut mentionner tout spécialement les chlorhydrates des composés selon la formule (I).

L'invention concerne tout particulièrement les dérivés de cyclopropylaniline répondant à la formule (II):

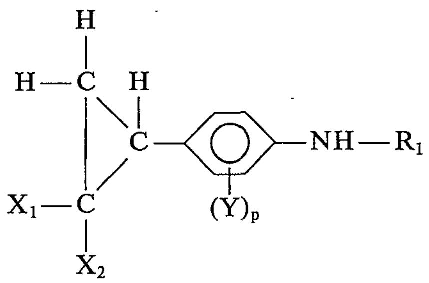

(II)

dans laquelle:

- $X_1$ représente un atome d'hydrogène ou d'halogène,
- $X_2$ représente un atome d'halogène,
- Y et $R_1$ ont même signification que dans la formule (I),
- p est égal à 0, 1 ou 2 (lorsque p est égal à 2, les Y peuvent être différents) ainsi que les sels que ces composés (II) peuvent former avec des acides (notamment les chlorhydrates).

De ce fait, l'invention concerne plus particulièrement, d'une part les compositions contenant comme matière active au moins un composé selon la formule (II) ou un sel de ce composé, et d'autre part, en tant que produits nouveaux, les composés selon la formule (II) et leurs sels, à l'exclusion des composés connus mentionnés plus haut.

Parmi les dérivés de cyclopropyl-4 aniline selon la formule (II), l'invention concerne de préférence, d'une part les composés pour lesquels (Y)p représente un atome d'halogène placé en position 3 sur le noyau benzénique, ainsi que les sels de ces composés, et d'autre part, les compositions contenant comme matière active ces composés ou sels.

Les composés selon la formule (I) peuvent être obtenus à partir des cyclopropylacétophénones de formule (III):

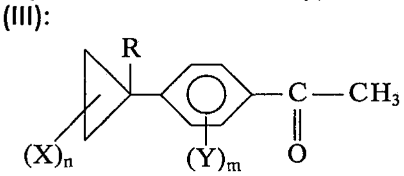

(III)

dans laquelle R, X, Y, n et m ont même signification que dans la formule (I), selon un procédé comprenant plusieurs ou la totalité des étapes suivantes:

Etape a (oximation)

Action d'un sel d'hydroxylamine, en présence de soude, sur la cyclopropylacétophénone de formule (III), pour donner un cyclopropylacétophénoxime (IV), selon la réaction:

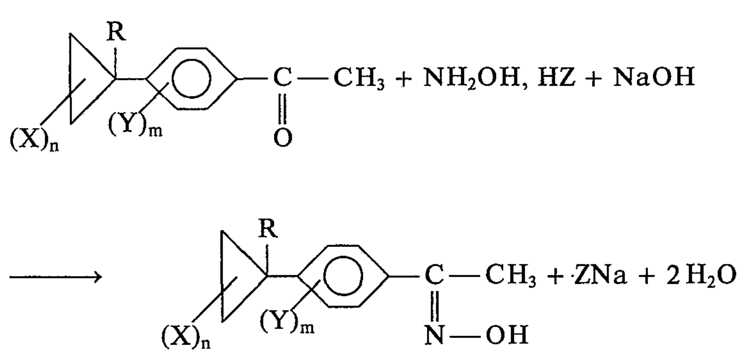


(IV)

dans laquelle X, Y, R, m et n ont la même signification que dans la formule I et Z représente un anion mono ou divalent d'un acide fort tel qu'un anion chlorure ou sulfate.

La réaction s'effectue avantageusement en coulant une solution aqueuse de soude dans une solution hydroorganique contenant du chlorhydrate d'hydroxylamine et la cyclopropylacétophénone à une température comprise entre 60 et 100° C environ.

Etape b (transposition)

Transformation du cyclopropylacétophénoxime (IV) résultant de l'étape »a« en un cyclopropylacétanilide (V), par transposition de Beckmann, selon le schéma suivant:

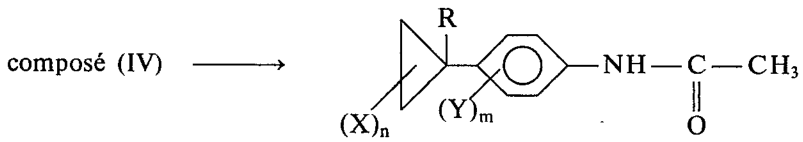

(V)

dans lequel R, X, Y, m et n ont la même signification que dans la formule (I). Cette transformation s'effectue selon les conditions généralement utilisables pour la transposition de Beckmann telles que décrites notamment dans »Organic Reactions II chapitre 1«.

Avantageusement, elle s'effectue par action d'un acide fort concentré tel que l'acide sulfurique ou l'acide polyphosphorique, à une température comprise entre 100 et 150° C environ et de préférence entre 120 et 130° C.

Etape c (hydrolyse)

Hydrolyse du cyclopropylacétanilide (V) résultant de l'étape »b« pour donner la cyclopropylaniline (VI) selon la réaction:

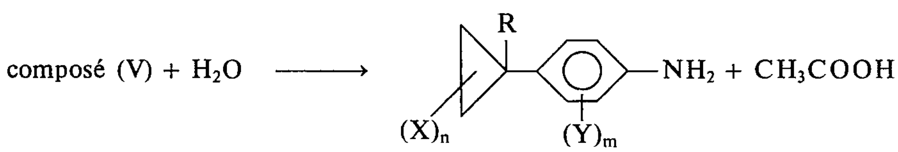

(VI)

dans laquelle R, X, Y, m et n ont la même signification que dans la formule (I).

Elle s'effectue par simple chauffage, après dilution, par l'eau, du mélange réactionnel résultant de l'étape »b«, à une température comprise entre 100 et 150° C environ et de préférence voisine de 130° C.

Etape d (alcoylation)

Alcoylation de la cyclopropylaniline (VI) résultant de l'étape C pour donner le composé selon la formule (I) dans laquelle $R_1$ représente un radical alcoyle.

Cette alcoylation peut s'effectuer selon une méthode analogue à celle décrite dans »Organic Syntheses — Collective Volume IV, p. 240« (dans le cas de la préparation de la N-méthyl-p-chloroaniline et de la N-éthyl-p-chloroaniline à partir de la p-chloroaniline), en faisant réagir un orthoformiate d'alcoyle de formule:

$$(R_1-O)_3-CH$$

dans laquelle $R_1$ représente un radical alcoyle comportant de 1 à 6 atomes de carbone, sur la cyclopropylaniline (VI), pour donner le cyclopropylformanilide de formule:

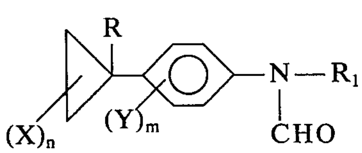

dans laquelle $R_1$ a même signification que précédemment et X, Y, R, m et n ont même signification que dans la formule I, puis en hydrolysant le cyclopropylformanilide ainsi obtenu.

L'action de l'orthoformiate d'alcoyle sur la cyclopropylaniline s'effectue avantageusement en présence d'un acide, à une température comprise entre 100 et 250°C environ. L'hydrolyse du cyclopropylformanilide s'effectue en chauffant à reflux le mélange réactionnel en présence d'un acide.

En fin de réaction, les produits formés peuvent être isolés par tout moyen en soi connu, tel que, par exemple, par filtration, par distillation du solvant, ou par recristallisation des produits formés, dans le milieu réactionnel, puis, si nécessaire, ils peuvent être purifiés selon les méthodes usuelles.

Si l'on se réfère au procédé décrit plus haut, on observera que les composés selon la formule I, pour lesquels $R_1$ représente l'atome d'hydrogène sont obtenus en effectuant les étapes successives a, b et c sans effectuer l'étape d. Les composés pour lesquels $R_1$ représente un radical alcoyle sont obtenus en effectuant de plus l'étape d, après les étapes successives a, b et c.

Avantageusement, les composés selon la formule I peuvent être transformés en sels, par traitement au moyen d'un acide approprié tel que par exemple l'acide chlorhydrique, l'acide sulfurique, l'acide nitrique, l'acide phosphoreux etc...

Parmi ces sels, les chlorhydrates de formule:

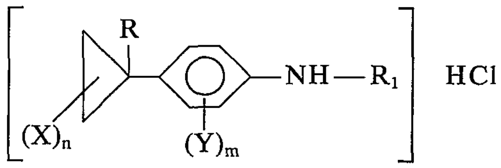

(VII)

sont particulièrement avantageux du fait qu'ils présentent en général une meilleure solubilité dans l'eau que les composés de formule I dont ils découlent. De ce fait, ces composés sous forme de chlorhydrates sont particulièrement avantageux pour la préparation de compositions à usage agricole destinées à être diluées par de l'eau. (Dans la formule VII, les différents substituants ont même signification que dans la formule I).

Le produit de départ utilisé pour la mise en oeuvre du procédé de l'invention peut être préparé comme indiqué ci-après:

- La cyclopropylacétophénone (III) pour laquelle m est égal à zéro peut être préparée par action du chlorure d'acétyle sur un cyclopropylbenzène. Certaines de ces cyclopropylacétophénones ont été décrites dans la littérature, notamment la cyclopropyl-4 acétophénone et la (dichloro-2,2 cyclopropyl)-4 acétophénone, décrites dans »Bulletin of the Chemical Society of Japan, volume 46, No 1, janvier 1973, p. 204–209«.
- La cyclopropylacétophénone (III) pour laquelle m est différent de zéro peut être préparée par action d'un halogène sur la cyclopropylacétophénone (III) non halogénée sur le noyau phényle. Cette halogénation peut s'effectuer en milieu anhydre, dans un solvant organique inerte, en présence d'un catalyseur du type acide de Lewis tel que par exemple $AlCl_3$ et $FeCl_3$.

Les exemples ci-après illustrent l'invention sans toutefois la limiter. La structure des composés décrits a été confirmée par spectrométrie infra-rouge et/ou par spectrométrie de résonance magnétique nucléaire (RMN), les spectres ayant été réalisés à 60 mégahertz, dans le DMSO, avec l'hexaméthylène-disiloxane comme référence interne.

## Exemple 1

### Préparation de la (dichloro-2,2 cyclopropyl)-4 chloro-3 aniline (composé No 1)

98,1 g de chlorure d'acétyle sont coulés goutte à goutte dans une suspension de 167 g (1,25 mole) de chlorure d'aluminium dans 350 ml de dichloréthane anhydre à une température comprise entre 0 et 5°C. Puis 187 g (1,0 mole) de dichloro-2,2 cyclopropyl-benzène sont coulés goutte à goutte à la même température en 2 heures.

Le mélange est agité à froid jusqu'à fin du dégagement gazeux. Après retour à température ambiante, le mélange réactionnel est versé sur 1 kg de glace et 50 ml d'acide chlorhydrique concentré.

Après 30 mn d'agitation, la phase organique est décantée et la phase aqueuse extraite trois fois par 100 ml de chlorure de méthylène puis les phases organiques réunies sont lavées par 200 ml d'eau puis par 200 ml de solution saturée de bicarbonate de sodium, séchées sur sulfate de sodium anhydre, évaporées et distillées sous 0,002 torr et on recueille ainsi 197,8 g de (dichloro-2,2 cyclopropyl) acétophénone (rendement 86%).

Le dichloro-2,2 cyclopropylbenzène utilisé comme matière de départ a été préparé selon le procédé décrit par Tetrahedron Letters 1969, p. 4569 et Chem. Ber. 108, 2803–2808 (1975).

Une solution de 96,0 g (0,42 mole) de (dichloro-2,2 cyclopropyl)-4 acétophénone dans 75 ml de

dichloréthane anhydre est coulée goutte à goutte dans une dispersion de 140 g de chlorure d'aluminium (1,05 mole) dans 75 ml de dichloréthane à une température comprise entre 0 et 5° C. Puis 33,0 g (0,46 mole) de chlore gazeux sont dissous dans le mélange en 45 minutes en maintenant toujours la température entre 0 et 5° C. Le mélange réactionnel est alors coulé sur 700 g de glace et 25 ml d'acide chlorhydrique concentré, décanté et la phase organique est lavée deux fois par 100 ml de solution de bicarbonate de soude à 10%, séchée sur sulfate de sodium et évaporée.

Le produit brut est recristallisé deux fois dans l'hexane et on obtient ainsi 53,0 g de (dichloro-2,2 cyclopropyl)-4 chloro-3 acétophénone fondant à 74° C: rendement: 48%.

Une solution de 26,4 g (0,66 mole) de soude dans 50 ml d'eau est coulée goutte à goutte en 15 minutes dans une solution hydroalcoolique (500 ml d'éthanol + 100 ml d'eau) de 46 g (0,66 mole) de chlorhydrate d'hydroxylamine et de 158,1 g de (dichloro-2,2 cyclopropyl)-4 chloro-3 acétophénone portée à l'ébullition.

La coulée terminée, le mélange réactionnel est maintenu à l'ébullition pendant 1 heure puis l'éthanol est évaporé sous vide et l'oxime précipite.

Il est essoré, lavé à l'eau et séché. Le composé brut est recristallisé dans un mélange hexane/cyclohexane et on obtient ainsi 119,0 g de (dichloro-2,2 cyclopropyl)-4 chloro-3 acétophénoxime fondant à 102° C (rendement 71,2%).

58,2 g de (dichloro-2,2 cyclopropyl)-4 chloro-3 acétophénoxime sont solubilisés dans 55 ml d'acide sulfurique concentré à une température comprise entre 10 et 15° C.

La solution visqueuse obtenue est ensuite coulée goutte à goutte dans 18 ml d'acide sulfurique concentré porté à 120 – 125° C à une vitesse telle que la température se maintienne entre 120 et 130° C sans apport extérieur de calories.

Une fois l'addition termnée, cette température est maintenue pendant 10 minutes par chauffage, puis le milieu est refroidi jusque vers 60° C, additionné de 73 ml d'eau et porté à l'ébullition pendant 2 heures. Par refroidissement sous agitation, le sulfate d'aniline précipite. Il est filtré, lavé à l'eau puis traité par 70 ml de soude 5N et 100 ml de chlorure de méthylène.

La solution chlorométhylénique est lavée à l'eau puis séchée sur sulfate de sodium anhydre. Après évaporation du solvant, l'huile noire obtenue (36,5 g) cristallise. Elle est recristallisée dans un mélange éther de pétrole (90%), cyclohexane (10%). On obtient ainsi 28,0 g de (dichloro-2,2 cyclopropyl)-4 chloro-3 aniline fondant à 66,5° C; rendement 66,5%.

Composition élémentaire

| | calculée | trouvée |
|---|---|---|
| C % | 45,67 | 45,97 |
| H % | 3,38 | 3,27 |
| N % | 5,92 | 5,81 |
| Cl % | 45,03 | 44,62 |

Exemple 2

Préparation de la (dichloro-2,2 cyclopropyl)-4 aniline (composé No 2)

On opère comme à l'exemple précédent à partir des mêmes matières premières si ce n'est que la (dichloro-2,2 cyclopropyl)-4 acétophénone résultant de l'acétylation n'est pas soumise à chloration, mais directement transformée par oximation, transposition et hydrolyse, selon les conditions indiquées dans cet exemple.

On obtient ainsi la (dichloro-2,2 cyclopropyl)-4 aniline fondant à 62,1° C.

Composition élémentaire

| | calculée | trouvée |
|---|---|---|
| C % | 53,49 | 53,86 |
| H % | 4,49 | 4,65 |
| N % | 6,93 | 6,74 |
| Cl % | 35,08 | 35,30 |

## Exemples 3 et 4

En opérant selon la méthode décrite dans les exemples 1 et 2, à partir des matières de départ appropriées, les composés suivants ont été préparés:

- (dichloro-2,2 cyclopropyl)-4 bromo-3 aniline (composé No 3), obtenu avec un rendement de 13%, sous forme d'un liquide brun clair,
- (dibromo-2,2 cyclopropyl)-4 aniline (composé No 4), obtenu avec un rendement de 6,4% sous forme d'un liquide brun rouge.

## Exemple 5

### Préparation de la (dichloro-2,2 cyclopropyl)-4 chloro-3 N-méthyl-aniline (composé No 5)

On opère à partir de la (dichloro-2,2 cyclopropyl)-4 chloro-3 aniline obtenue dans l'exemple No 1.

Dans un ballon tricol de 250 ml muni d'une colonne vigreux, d'un réfrigérant descendant et d'un thermomètre, on place 47,3 g (0,2 mole) de (dichloro-2,2 cyclopropyl)-4 chloro-3 aniline, 31,8 g (0,3 mole) d'orthoformiate de triméthyle et 0,8 g d'acide sulfurique concentré. On chauffe le mélange réactionnel à 115–120°C et on distille l'éthanol formé. On chauffe ensuite à 165°C pendant 45 mn. Après refroidissement, le produit brut est purifié par chromatographie en phase liquide sur silice. On obtient 32,3 g de N-Méthyl (dichloro-2,2 cyclopropyl)-4 chloro-3 formanilide fondant à 90°C.

Ce dernier est placé dans un ballon de 250 ml avec 100 ml d'acide chlorhydrique à 10% et chauffé à reflux pendant 2 heures. Après refroidissement à 10°C, on neutralise le mélange réactionnel avec de la potasse à 15%. On extrait le produit à l'éther éthylique, lave à l'eau et sèche sur sulfate de sodium anhydre. Après évaporation de l'éther éthylique, le produit brut est chromatographié en phase liquide sur silice.

On obtient ainsi 20,7 g de (dichloro-2,2 chyclopropyl)-4 chloro-3-N-méthyl aniline ayant un indice de refraction $n_D^{22}$ = 1,5878. Rendement = 71,6%.

## Exemple 6

En opérant selon la méthode décrite à l'exemple 5, à partir des matières premières convenables, on obtient la (dichloro-2,2 cyclopropyl)-4 chloro-3-N-éthyl-aniline (composé No 6) ayant un indice de réfraction $n_D^{22}$ = 1,57810. Rendement de 63%.

## Exemple 7

### Préparation du chlorhydrate de (dichloro-2,2 cyclopropyl)-4 chloro-3 aniline (composé No 7)

On opère à partir de la (dichloro-2,2 cyclopropyl)-4 chloro-3 aniline dont la préparation est décrite dans l'exemple No 1.

On introduit, dans un ballon de 250 ml surmonté d'un réfrigérant ascendant, 4 g (0,017 mole) de (dichloro-2,2 cyclopropyl)-4 chloro-3 aniline, 10 ml d'éthanol et 120 ml d'acide chlorhydrique à 5%. On chauffe à reflux pendant 30 mn. Après refroidissement, le chlorhydrate précipite. On filtre et sèche à 50°C et sous vide (6 mm Hg).

On obtient ainsi 2,7 g de chlorhydrate de (dichloro-2,2 cyclopropyl)-4 chloro-3 aniline fondant à 216°C, soit un rendement de 58,7%.

Composition élémentaire

| | calculée | trouvée |
|---|---|---|
| C % | 38,16 | 38,72 |
| H % | 3,53 | 3,57 |
| N % | 4,95 | 4,90 |
| Cl % | 25,09 | 25,86 |

Exemples 8 à 10

En opérant selon la méthode décrite à l'exemple 7, respectivement à partir des composés No 3, 2 et 5, les composés suivants ont été préparés:

– Composé No 8: chlorhydrate de (dichloro-2,2-cyclopropyl)-4-bromo-3-aniline.
Point de fusion: 214–215°C

Composition élémentaire

| | calculée | trouvée |
|---|---|---|
| C % | 34,02 | 33,08 |
| H % | 2,83 | 2,80 |
| N % | 4,41 | 4,35 |
| Cl % | 33,54 | 32,17 |

– Composé No 9: chlorhydrate de (dichloro-2,2-cyclopropyl)-4-aniline.
Point de fusion: supérieur à 250°C.

Composition élémentaire

| | calculée | trouvée |
|---|---|---|
| C % | 45,28 | 45,92 |
| H % | 4,19 | 3,80 |
| N % | 5,87 | 5,01 |
| Cl % | 44,65 | 44,52 |

– Composé No 10: chlorhydrate de (dichloro-2,2-cyclopropyl)-4-chloro-3-N-méthyl-aniline.
Point de fusion: 151°C.

Composition élémentaire

| | calculée | trouvée |
|---|---|---|
| C % | 41,81 | 42,14 |
| H % | 3,83 | 3,[illegible]5 |
| N % | 4,88 | 4,85 |
| Cl % | 49,47 | 49,25 |

Dans les essais décrits ci-après dans les exemples I à IV on entendra par solution soit une solution aqueuse lorsque la matière active est hydrosoluble, soit, dans le cas contraire, une dispersion ou émulsion aqueuse obtenue en diluant par de l'eau, respectivement, soit une poudre mouillable comprenant en poids 20% de matière active, soit un concentré émulsionnable contenant environ 200 g/l de matière active. Ces essais sont effectués soit en serre, soit en plein champ, en traitant les feuilles de plantes telles que haricot et soja par une solution dont la teneur en matière active à tester peut varier de 0,1 g/l à 20 g/l. On observe ensuite l'évolution biométrique et morphologique dans le temps, des plantes traitées et on la compare à celle de plantes témoins traitées dans les mêmes conditions par une solution ne contenant pas de matière active.

## Exemple I

### Essai en serre sur haricot (Phaseolus vulgaris) de variété Contender

On prépare une solution de la matière à tester, à la concentration voulue, en diluant par de l'eau un concentré émulsionnable ayant la composition suivante:

| | |
|---|---|
| — matière à tester | 206 g |
| — agent mouillant et émulsifiant (mélange 4/l d'un condensat d'oxyde d'éthylène/huile de ricin comprenant de 30 à 33 motifs oxyde d'éthylène et de dodécylbenzène sulfonate de sodium) | 102 g |
| — solvant (hydrocarbures aromatiques résultant de fraction de pétrole) | 692 g |

Dans des pots de 9 × 9 × 9 cm, remplis de terre agricole légère, on sème des graines de haricot que l'on recouvre ensuite par une couche de terre d'un demi centimètre d'épaisseur et les pots sont conservés en serre à température ambiante, sous 70% d'humiditié relative et arrosés en subirrigation.

Lorsque les plants de haricot ont atteint le stade deux feuilles primordiales développées et bourgeon terminal prêt à s'ouvrier, on traite leur feuillage par pulvérisation au moyen de la solution préparée précédemment, à raison d'environ 500 l/ha de solution, pour des doses de matière active allant de 2 à 8 kg/ha.

Les pots traités sont ensuite placés dans des bacs destinés à recevoir l'eau d'arrosage en subirrigation, puis maintenus pendant 35 jours, à température ambiante sous 70% d'humidité relative. Au bout de ces 35 jours, on observe que les plants traités par le composé No 1 à 8 kg/ha présentent une taille en moyenne égale à la moitié de celle des témoins, et qu'ils présentent de plus, par rapport aux témoins, un développement très important des pousses latérales provenant des bourgeons axilaires. Pour des doses de 2 et 4 kg/ha, les plants traités par le composé No 1 présentent une taille en moyenne égale respectivement à 80% et 40% de celle des témoins.

Les plants traités par les composés No 2, 5, 6, 7, 9 et 10, à 8 kg/ha présentent une taille en moyenne égale à la moitié de celle des témoins, ainsi que ceux traités à 4 kg/ha par les composés No 2, 6, 7, 8, 9 et 10.

Les plants traités par les composés No 5, 6, 7 et 9 à 2 kg/ha présentent une taille en moyenne égale à 75% de celle des témoins, ainsi que ceux traités à 4 kg/ha par le composé No 5 et à 1 kg/ha par les composés No 7 et 10.

## Exemple II

### Essai en serre sur soja (Glycine max) de variété Amsoy 71

On utilise une solution préparée en diluant par de l'eau le même concentré émulsionnable qu'à l'exemple I.

Dans des pots de 20 × 20 × 20 cm contenant en proportions égales, de la tourbe, du sable et de la terre végétale, on sème des graines de soja, préalablement inoculées par le rhizobium à raison de 1 graine par pot et on conserve les pots en serre comme dans l'exemple 1, avec toutefois arrosage par la surface des pots.

Lorsque les plants de soja ont atteint le stade 3 feuilles trifoliées développées, soit environ 30 jours après la mise en place de l'essai, on traite les feuillages par pulvérisation comme à l'exemple I à raison de 500 l/ha de solution. Les pots traités sont ensuite maintenus 15 jours en serre, à température ambiante, sous 70% d'humidité relative.

Au bout de ces 15 jours, on observe que, les plants traités par le composé No 1 à la dose de 2 kg/ha des matière active, présentent en moyenne, par rapport au témoin, une augmentation de 50% du nombre des ramifications secondaires.

## Exemple III

### Essai de plein champ sur soja (Glycine max.) de variété Amsoy 71

Dans des parcelles de 12 $m^2$ chacune, on sème le 15 mai des graines de soja préalablement inoculées par le rhizobium.

Lorsque les plants de soja sont au stade troisième feuille vraie étalée (soit environ un mois après la mise en place de l'essai), on traite ces plants par pulvérisation au moyen d'une solution préparée en diluant par de l'eau, à la concentration désirée, le même concentré émulsionnable qu'à l'exemple I et en appliquant cette solution à raison de 3 litres par parcelle (2500 l/ha).

Pour chaque dose, on effectue deux essais dans des parcelles distinctes. Trois mois et demi après le

traitement, on dénombre, dans le cas des parcelles traitées et des parcelles témoins, d'une part le nombre de ramifications fructifères (c'est-à-dire porteuses de gousses) et, d'autre part le nombre de gousses formées sur les tiges principales et sur les ramifications fructifères et on calcule les valeurs moyennes rapportées à un plant de soja.

Dans ces conditions on observe que, à la dose de 2 kg/ha de matière active, le composé No 1 donne les résultats suivants (valeurs exprimées pour un plant de soja):

| | Composé No 1 | Témoin |
|---|---|---|
| – Nombre de ramifications fructifères | 3,7 | 1,6 |
| – Nombre de gousses sur la tige principale | 25,4 | 25,2 |
| – Nombre de gousses sur les ramifications fructifères | 14,0 | 8,7 |

Exemple IV

Essai de plein champ sur soja (Glycine max) de variété Amsoy 71

Dans une parcelle de 3 x 10 m, on sème fin juillet des graines de soja, selon deux rangées parallèles de 10 m de long chacune, espacées entre elles de 0,50 m.

On utilise une solution préparée en diluant par de l'eau, à la concentration voulue, le même concentré émulsionnable qu'à l'exemple I.

Le 30 Août, lorsque les plants de soja sont au stade six feuilles trifoliées étalées, on traite les plants de la première rangée au moyen de la solution préparée précedemment, de façon à ce que la dose de matière active appliquée sur la première rangée soit de 1 kg/ha, la deuxième rangée n'étant soumise à aucun traitement de façon à être utilisée comme témoin. Les résultats observés sont indiqués ci-dessous:

| | Témoin | Composé No 1 |
|---|---|---|
| – Dose de matière active | 0 | 1 Kg/ha |
| – Nombre de ramifications (100 plants) | 162 | 195 |
| – Nombre de gousses (100 plants) | 3 111 | 3 749 |
| – Poids de grains récoltés (100 plants) | 1 436 g | 1 603 g |
| – Poids de 1000 grains | 162 g | 156 g |

Ces résultats montrent que le traitement effectué au moyen du composé No 1, selon les conditions indiquées précédemment entraîne à la fois une augmentation du nombre des ramifications fructifères et du nombre de gousses des plants de soja.

Ils montrent bien les propriétés remarquables des composés selon l'invention qui peuvent donc être utilisés sur toutes sortes de plantes monodicotylédones (notamment graminées et dicotylédones), comme celles des grandes cultures, cultures industrielles, fruits, légumes, plantes d'ornemant, plantes médicinales et à parfums, en vue d'augmenter les rendements, favoriser la ramification, modifier le port, réduire la taille pour obtenir des plantes plus ramassées etc. . .

Pour leur emploi dans la pratique, les composés selon l'invention sont rarement utilisés seuls. Le plus souvent, ils font partie de compositions qui comprennent, en général, en plus de la matière active selon l'invention, un support et/ou un agent tensioactif.

De façon générale, ces compositions contiennent de 0,001 à 95% en poids de matière active, un support acceptable en agriculture et/ou de 0 à 20% d'un agent tensioactif.

Le terme »support« au sens de la présente description désigne une matière, organique ou minérale, naturelle ou synthétique, avec laquelle la matière active est associée pour faciliter son application sur

la plante, sur des graines ou sur le sol, ou son transport, ou sa manipulation. Le support peut être solide (par exemple argiles, silicates naturels ou synthétiques, carbonates de calcium ou de magnésium, sulfate de calcium) ou liquide (par exemple eau, alcools, cétones, éthers-oxydes, esters, hydrocarbures aromatiques, hydrocarbures halogènés, fractions de pétrole).

L'agent tensioactif (ou agent de surface), au sens de la présente description, peut être un agent mouillant, dispersant ou émulsifiant, pouvant être ionique ou non ionique. On peut citer, par exemple, des non ioniques, comme les condensats d'oxyde d'éthylène avec les alcools gras, acides gras, amides ou amines grasses ou les alkylphénols, les esters d'acides gras, et du sorbitol, dérivés du saccharose, des anioniques, comme les sels d'acides lignosulfoniques, d'acides alkylarylsulfoniques, d'alcoyl sulfosuccinates et sulfosuccinamates, des dérivés d'aminoacides, des cationiques comme des acétates d'alcoylamines ou d'imidazoline, des amphotères comme les alcoylbétaïnes ou sulfobétaïnes.

En plus de la matière active, du support et de l'agent tensioactif, les compositions selon l'invention peuvent contenir d'autres additifs tels que des agents dispersants non tensioactifs, des peptisants, colloides protecteurs, des épaississants, des adhésifs augmentant la résistance à la pluie, des stabilisants, des agents conservateurs, des inhibiteurs de corrosions, des colorants, des séquestrants, des anti-mousses, anti-mottants, anti-gels etc...

Les compositions selon l'invention peuvent être préparées sous la forme de poudres mouillables, de poudres solubles, de poudres pour poudrage, de granulés, de solutions, de concentrés émulsionnables, d'émulsions, suspensions concentrées ou de suspensions et de dispersions.

Les poudres mouillables sont habituellement préparées par pré-mélange de la matière active avec un support solide et des agents de mouillage et de dispersion, sous forme de poudres, de manière qu'elles contiennent de 10 à 95% en poids de la matière active et de 0,5 à 20% en poids d'agents de surface. Ce pré-mélange est ensuite broyé soit dans un broyeur, par exemple à broches équipé d'un sélecteur à poudre, soit dans un broyeur »Air Jet«, ce dernier appareil étant préféré pour les matières actives à bas point de fusion.

A titre d'exemples, voici la composition de plusieurs poudres mouillables selon l'invention; les pourcentages étant exprimés en poids:

| | | |
|---|---|---|
| — | Composé No 1 | 25,0% |
| | alcoyl sulfosuccinate de sodium (mouillant) | 2,0% |
| | sel alcalin d'un naphtalène sulfonate condensé (dispersant) | 7,0% |
| | argile kaolinique | 66,0% |
| — | Composé No 2 | 50,0% |
| | alkylnaphtalène sulfonate de sodium (mouillant) | 3,0% |
| | lignosulfonate alcalin (dispersant) | 10,0% |
| | argile kaolinique | 37,0% |
| — | Composé No 1 | 50,0% |
| | polyéthoxyéther d'alcool gras (mouillant) | 1,4% |
| | lignosulfonate d'alcalino-terreux (dispersant) | 10,0% |
| | silice fossile | 38,6% |
| — | Composé No 1 | 50,0% |
| | alkylnaphtalène sulfonate de sodium (mouillant) | 4,0% |
| | sel alcalin d'acides sulfoniques condensés (dispersant) | 3,0% |
| | argile kaolinique | 20,0% |
| | silice fossile | 23,0% |
| — | Composé No 1 | 60,0% |
| | polyéthoxyéther d'alkylphénol (mouillant) | 2,0% |
| | sel alcalin d'un naphtalène sulfonate condensé (dispersant) | 5,0% |
| | argile kaolinique | 33,0% |

Les poudres solubles dans l'eau sont habituellement obtenues par mélange de 20 à 95% en poids de matière active, de 0 à 10% d'une charge antimottante et de 0 à 1% d'un agent mouillant, le reste étant constitué par une charge hydrosoluble principalement un sel.

Voici un exemple de composition d'une poudre hydrosoluble:

| | | |
|---|---|---|
| — | matière active (composé No 1) | 80,0% |
| — | agent mouillant anionique (alcoyl naphtalène sulfonate alcalin) | 0,5% |
| — | silice antimottante | 4,0% |
| — | sulfate de sodium (charge soluble) | 15,5% |

Les poudres pour poudrages peuvent être préparées comme les poudres mouillables mais sans les tensioactifs, dispersants, colloïdes protecteurs inutiles dans ces formulations. Dans ce cas, le support solide est choisi spécialement pour faciliter le broyage, absorber si la matière active est liquide et garantir la fluence.

Les granulés et micro-granulés, généralement à faible titre de matière active, peuvent être pré-fabriqués puis imprégnés ou fabriqués dans la masse par exemple par atomisation ou par extrusion. Dans ce dernier cas, ils nécessitent l'adjonction de liants et de produits hygroscopiques pour assurer leur délitage rapide au sol. Ils contiennent en général de 0,5 à 25% en poids de matière active, de 0 à 10% en poids d'additifs comme des stabilisants, des agents de modification à libération lente, des liants et des solvants.

Les concentrés émulsionnables contiennent la matière active dissoute dans un solvant (généralement un hydrocarbure aromatique) et en plus du solvant et quand c'est nécessaire, un solvant auxiliaire ou cosolvant pouvant être une cétone, un ester, un éther-oxyde, etc. Ils contiennent de 10 à 60% en poids/volume de matières actives, de 2 à 20% en poids/volume d'agent émulsifiant et peuvent être préparés, par exemple, par dissolution de matière active, dans le solvant ou dans le mélange de solvants et émulsifiant, dans une cuve équipée d'une bonne agitation et d'une circulation de fluide pour le chauffage ou le refroidissement.

A titre d'exemple, voici la composition de deux concentrés émulsionnables selon l'invention:

| | |
|---|---|
| — matière active (composé No 1) | 200 g |
| — alkylarylsulfonate de calcium | 20 g |
| — nonylphénol oxyéthyléné à 9 motifs oxydes d'éthylène | 80 g |
| — solvant (hydrocarbures aromatiques provenant de fraction de pétrole) | q.s.p. 1 litre (soit 667 g) |

| | |
|---|---|
| — matière active (composé No 1) | 400 g |
| — dodécylbenzène sulfonate de sodium | 24 g |
| — nonylphénol oxyéthylène à 10 molécules d'oxyde d'éthylène | 16 g |
| — cyclohexanone | 200 g |
| — solvant (hydrocarbures aromatiques) | q.s.p. 1 litre |

Les suspensions concentrées appelées »flowables«, également applicables en pulvérisation après dilution dans l'eau, sont préparées de manière qu'on obtienne un produit fluide, stable, ne se déposant pas. Elles contiennent de 10 à 50% en poids de matière active, de 0,5 à 15% en poids d'agents tensioactifs, de 0,5 à 10% en poids de colloïdes protecteurs ou agents antisédiments, de 0 à 10% d'additifs divers, comme des anti-mousses, épaississants, stabilisants, adhésifs, anti-gels et comme support de l'eau ou un liquide organique dans lequel la matière active est insoluble ou un mélange des deux. Certaines matières solides organiques ou des sels minéraux peuvent être dissous dans le support pour retarder la sédimentation ou comme anti-gel pour l'eau.

Ces suspensions concentrées peuvent être préparées, par exemple, par dispersion de la matière active pré-broyées dans un support liquide pouvant être de l'eau, une huile végétale ou minérale ou le mélange émulsionné des deux contenant les ingrédients auxiliaires comme l'agent de viscosité, le conservateur et l'antigel, puis broyage de la dispersion dans un broyeur à billes.

Des dispersions et émulsions aqueuses, par exemple des compositions obtenues en diluant à l'aide d'eau une poudre mouillable ou un concentré émulsionnable selon l'invention, sont comprises dans le cadre général de la présente invention. Ces émulsions peuvent être du type eau-dans-l'huile ou du type huile-dans-l'eau, et elles peuvent avoir une consistance épaisse comme celle d'une »mayonnaise«.

Toutes ces compositions peuvent être apportées aux plantes par diverses méthodes comme la pulvérisation sur la partie aérienne des plantes, le trempage des graines, des plantes, des mottes, des racines ou des fruits, l'arrosage du sol, l'injection à la plante etc...

La présente invention concerne enfin un procédé pour modifier la croissance des plantes (c'est-à-dire pour modifier leur physiologie de diverses manières) selon lequel on applique sur les plantes une quantité efficace d'au moins un composé selon la formule (I), ou un sel d'un de ces composés. La dose de matière active à utiliser peut varier selon différents facteurs tels que le type de culture à traiter, son stade de développement, les conditions climatiques, la nature du terrain etc. En pratique, les traitements selon l'invention sont effectués en appliquant sur lesdites cultures des doses de matière active pouvant aller de 0,05 kg/ha à 10 kg/ha.

**Revendications pour les Etats contractants: BE, CH, DE, FR, GB, IT, LU, LI, NL**

1. Composition régulatrice de la croissance des plantes, caractérisée en ce qu'elle contient comme matière active au moins:

un dérivé de cyclopropylaniline de formule:

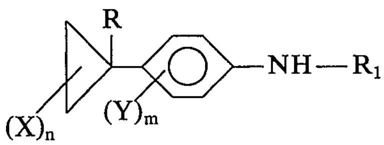

dans laquelle:

- R représente un atome d'hydrogène ou un radical alcoyle contenant de 1 à 4 atomes de carbone,
- X et Y, identiques ou différents, représentent chacun un atome d'halogène,
- $R_1$ représente un atome d'hydrogène ou un radical alcoyle contenant de 1 à 6 atomes de carbone,
- n est un nombre entier égal à 0, 1 ou 2, étant entendu que, lorsque n est supérieur à 1, les atomes X peuvent être identiques ou différents,
- m est un nombre entier égal à 0, 1, 2, 3 ou 4, étant entendu que lorsque m est supérieur à 1, les atomes Y peuvent être identiques ou différents,

ou un sel de ce dérivé de cyclopropylaniline.

2. Composition selon la revendication 1, caractérisée en ce qu'elle contient comme matière active au moins:

un dérivé de cyclopropylaniline de formule:

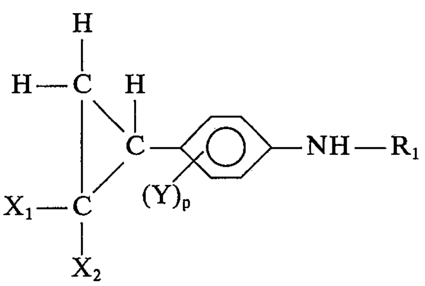

dans laquelle:

- $X_1$ représente un atome d'hydrogène ou d'halogène,
- $X_2$ représente un atome d'halogène,
- Y et $R_1$ ont même signification que dans la revendication 1,
- et p est égal à 0, 1 ou 2

ou un sel de ce dérivé de cyclopropylaniline.

3. Composition selon l'une des revendications 1 et 2, caractérisée en ce qu'elle contient de 0,001 à 95% en poids, de matière active ainsi qu'un support et/ou agent tensioactif.

4. Dérivé de la cyclopropylaniline, caractérisé en ce qu'il répond à la formule:

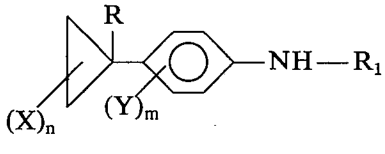

dans laquelle R, X, Y, $R_1$, m et n ont même signification que dans la revendication 1, et sels de ce dérivé de cyclopropylaniline formés par réaction avec un acide sous réserve que ce composé n'est ni la (dichloro-2,2 cyclopropyl)-4 aniline, ni la cyclopropyl-4 aniline.

5. Composé selon la revendication 4, caractérisée en ce qu'il répond à la formule:

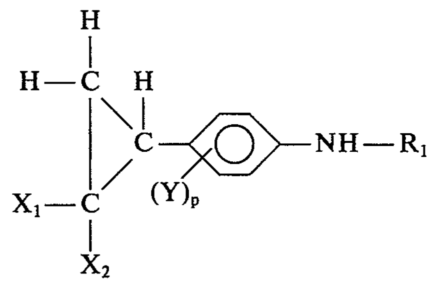

dans laquelle $X_1$, $X_2$, Y, $R_1$ et p ont même signification que dans la revendication 2 et les sels de ce composé.

6. Procédé de préparation d'un composé selon la revendication 4, caractérisé en ce qu'il comprend les étapes a, b, c et éventuellement l'étape d suivantes:

a action d'un sel d'hydroxylamine, en présence de soude, sur la cyclopropylacétophénone de formule:

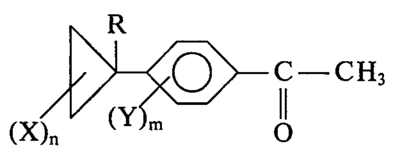

pour donner un cyclopropylacétophénoxime de formule:

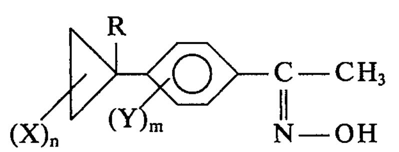

dans lesquelles X, Y, m, n et R ont même signification que dans la revendication 4.

b transposition du cyclopropylacétophénoxime résultant de l'étape a pour donner un cyclopropylacétanilide de formule:

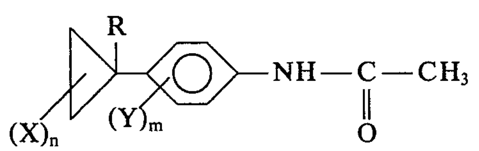

dans laquelle X, Y, m, n et R ont même signification que dans l'étape a.

c hydrolyse du cyclopropylacétanilide résultant de l'étape b, pour donner une cyclopropylaniline de formule:

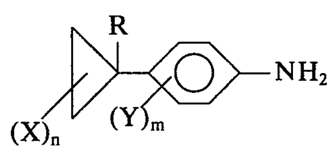

dans laquelle X, Y, m, n et r ont la même signification que dans l'étape b.

d alcoylation du composé résultant de l'étape c pour donner son dérivé N-alcoylé.

7. Procédé selon la revendication 6, caractérisé en ce que l'étape a est effectuée en solution hydroorganique, à une température comprise entre 60 et 100°C, en utilisant, comme sel d'hydroxylamine, le chlorhydrate ou le sulfate d'hydroxylamine.

8. Procédé selon la revendication 6, caractérisé en ce que l'étape b est effectuée par action d'un acide fort concentré, à une température comprise entre 100 et 150°C.

9. Procédé selon la revendication 6, caractérisé en ce que l'étape c est effectuée en diluant par de l'eau le mélange réactionnel résultant de l'étape b, puis en le chauffant à une température comprise entre 100 et 150°C.

10. Procédé pour modifier la croissance des plantes, caractérisé en ce que l'on applique sur ces

plantes une quantité efficace d'au moins un composé de formule:

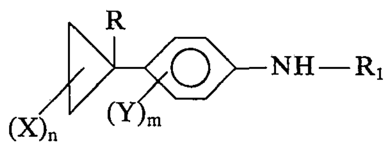

dans laquelle X, Y, m, n, R et $R_1$ ont même signification que dans la revendication 1, ou d'un sel d'un de ces composés.

**Revendications pour l'Etat contractant: AT**

1. Composition régulatrice de la croissance des plantes, utilisable en agriculture, caractérisée en ce qu'elle contient comme matière active au moins:

un dérivé de cyclopropylaniline de formule:

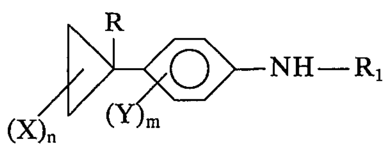

dans laquelle:

- R représente un atome d'hydrogène ou un radical alcoyle contenant de 1 à 4 atomes de carbone,
- X et Y, identiques ou différents, représentent chacun un atome d'halogène,
- $R_1$ représente un atome d'hydrogène ou un radical alcoyle contenant de 1 à 6 atomes de carbone,
- n est un nombre entier égal à 0, 1 ou 2, étant entendu que, lorsque n est supérieur à 1, les atomes X peuvent être identiques ou différents,
- m est un nombre entier égal à 0, 1, 2, 3 ou 4, étant entendu que lorsque m est supérieur à 1, les atomes Y peuvent être identiques ou différents,

ou un sel de ce dérivé de cyclopropylaniline.

2. Composition selon la revendication 1, caractérisée en ce qu'elle contient comme matière active au moins:

un dérivé de cyclopropylaniline de formule:

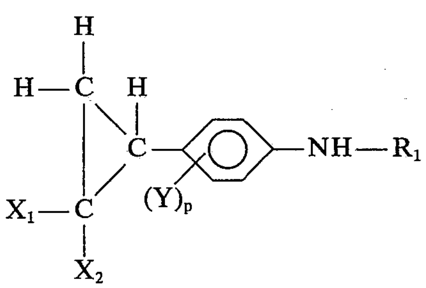

dans laquelle:

- $X_1$ représente un atome d'hydrogène ou d'halogène,
- $X_2$ représente un atome d'halogène,
- Y et $R_1$ ont même signification que dans la revendication 1,
- et p est égal à 0, 1 ou 2

ou un sel de ce dérivé de cyclopropylaniline.

3. Composition selon l'une des revendications 1 et 2, caractérisée en ce qu'elle contient de 0,001 à 95% en poids, de matière active ainsi qu'un support et/ou agent tensioactif.

4. Procédé de préparation d'un dérivé de cyclopropylaniline répondant à la formule:

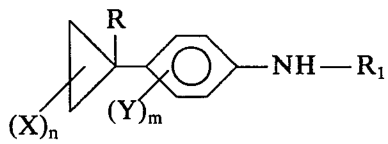

dans laquelle R, X, Y, $R_1$, m et n ont même signification que dans la revendication 1, caractérisé en ce qu'il comprend les étapes a, b, c et éventuellement l'étape d suivantes:

a action d'un sel d'hydroxylamine, en présence de soude, sur la cyclopropylacétophénone de formule:

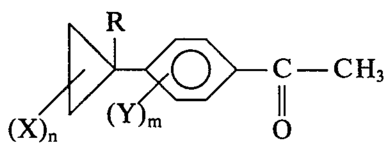

pour donner un cyclopropylacétophénoxime de formule:

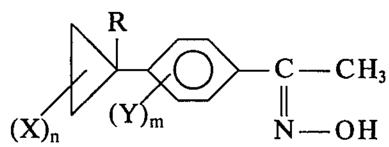

dans lesquelles X, Y, m, n et R ont même signification que dans la revendication 1.

b transposition du cyclopropylacétophénoxime résultant de l'étape a pour donner un cyclopropylacétanilide de formule:

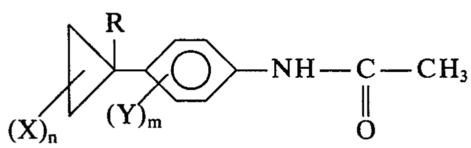

dans laquelle X, Y, m, n et R ont même signification que dans l'étape a.

c hydrolyse du cyclopropylacétanilide résultant de l'étape b, pour donner une cyclopropylaniline de formule:

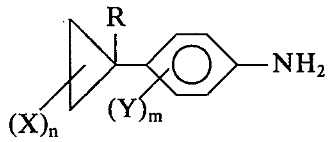

dans laquelle X, Y, m, n et r ont la même signification que dans l'étape b.

d alcoylation du composé résultant de l'étape c pour donner son dérivé N-alcoylé.

5. Procédé selon la revendication 4, caractérisé en ce que l'étape a est effectuée en solution hydroorganique, à une température comprise entre 60 et 100°C, en utilisant, comme sel d'hydroxylamine, le chlorhydrate ou le sulfate d'hydroxylamine.

6. Procédé selon la revendication 4, caractérisé en ce que l'étape b est effectuée par action d'un acide fort concentré, à une température comprise entre 100 et 150°C.

7. Procédé selon la revendication 4, caractérisé en ce que l'étape c est effectuée en diluant par de l'eau le mélange réactionnel résultant de l'étape b, puis en le chauffant à une température comprise entre 100 et 150°C.

8. Procédé de préparation des sels du dérivé de cyclopropylaniline répondant à la formule:

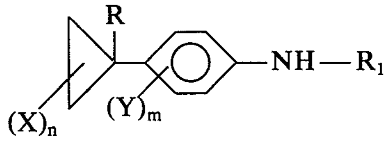

dans laquelle R, X, Y, $R_1$ et m ont même signification que dans la revendication 1, caractérisé en ce qu'il consiste à traiter ce dérivé par un acide.

9. Procédé selon la revendication 8, caractérisé en ce que l'acide est l'acide chlorhydrique, l'acide sulfurique, l'acide nitrique ou l'acide phosphoreux.

10. Procédé pour modifier la croissance des plantes, caractérisé en ce que l'on applique sur ces plantes une quantité efficace d'au moins un composé de formule:

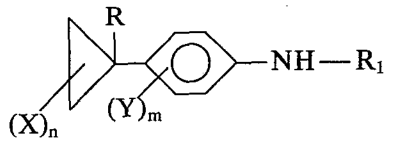

dans laquelle X, Y, m, n, R et $R_1$ ont même signification que dans la revendication 1, ou d'un sel d'un de ces composés.

**Patentansprüche für die Vertragsstaaten: BE, CH, DE, FR, GB, IT, LI, LU, NL**

1. Zusammensetzung für die Wachstumsregulierung von Pflanzen, dadurch gekennzeichnet, daß sie als aktives Material enthält wenigstens:

ein Cyclopropylanilinderivat der Formel

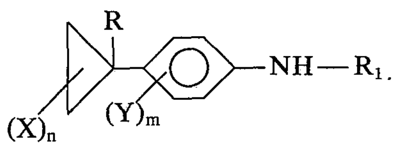

worin:

- R ein Wasserstoffatom oder einen Alkylrest mit 1 bis 4 Kohlenstoffatomen bedeutet,
- X und Y, welche identisch oder verschieden sind, jeweils ein Halogenatom bedeuten,
- $R_1$ ein Wasserstoffatom oder einen Alkylrest mit 1 bis 6 Kohlenstoffatomen bedeutet,
- n eine ganze Zahl gleich Null, 1 oder 2, ist, wobei, falls n größer als 1 ist, die Atome X identisch oder verschieden sein können,
- m eine ganze Zahl gleich Null, 1, 2, 3 oder 4 ist, wobei, falls m größer als 1 ist, die Atome Y identisch oder verschieden sein können,

oder ein Salz dieses Cyclopropylanilinderivats.

2. Zusammensetzung gemäß Anspruch 1, dadurch gekennzeichnet, daß sie als aktives Material enthält mindestens:

ein Cyclopropylanilinderivat der Formel

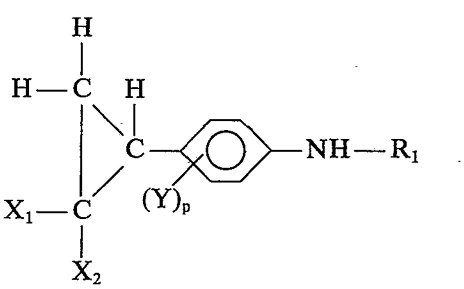

worin:

- $X_1$ ein Wasserstoff- oder Halogenatom bedeutet,
- $X_2$ ein Halogenatom bedeutet,
- Y und $R_1$ die gleiche Bedeutung wie in Anspruch 1 haben,
- und p Null, 1 oder 2 ist

oder ein Salz dieses Cyclopropylanilinderivats.

3. Zusammensetzung gemäß einem der Ansprüche 1 und 2, dadurch gekennzeichnet, daß sie von

0,001 bis 95 Gew.-% an aktivem Material sowie einen Träger und/oder ein oberflächenaktives Mittel enthält.

4. Derivat des Cyclopropylanilins, dadurch gekennzeichnet, daß es der Formel entspricht:

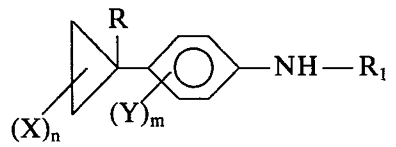

worin R, X, Y, $R_1$, n und m dieselbe Bedeutung wie in Anspruch 1 haben und Salze dieses Cyclopropylanilinderivats, gebildet durch Reaktion mit einer Säure mit der Ausnahme, daß diese Verbindung weder 4-(2,2-Dichlorcyclopropyl)-anilin noch 4-Cyclopropylanilin ist.

5. Verbindung gemäß Anspruch 4, dadurch gekennzeichnet, daß sie der Formel entspricht:

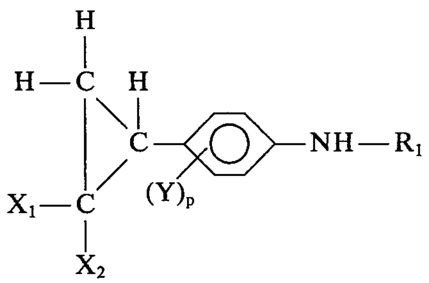

worin $X_1$, $X_2$, Y, $R_1$ und p dieselbe Bedeutung wie in Anspruch 2 haben und die Salze dieser Verbindung.

6. Verfahren zur Herstellung einer Verbindung gemäß Anspruch 4, dadurch gekennzeichnet, daß es die folgenden Stufen a, b, c und gegebenenfalls die Stufe d umfaßt:

a. Einwirkung eines Hydroxylaminsalzes in Gegenwart von Natriumhydroxyd auf Cyclopropylacetophenon der Formel:

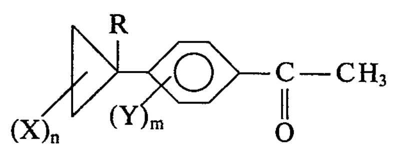

um ein Cyclopropylacetophenoxim der Formel

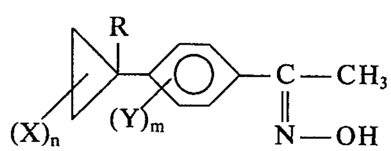

zu ergeben, worin X, Y, m, n und R dieselbe Bedeutung wie in Anspruch 4 haben.

b. Umlagerung des von der Stufe a resultierenden Cyclopropylacetophenoxims, um ein Cyclopropylacetanilid der Formel

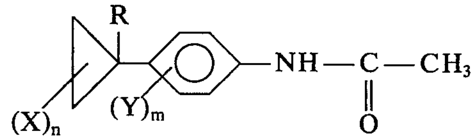

zu ergeben, worin X, Y, m, n et R dieselbe Bedeutung wie in Stufe a haben;

c. Hydrolyse des von der Stufe b resultierenden Cyclopropylacetanilids, um ein Cyclopropylanilin der Formel

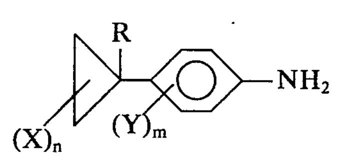

zu ergeben, worin X, Y, m, n und R dieselbe Bedeutung wie in Stufe b haben;

d. Alkylierung der von der Stufe c resultierenden Verbindung, um deren N-alkyliertes Derivat zu ergeben.

7. Verfahren gemäß Anspruch 6, dadurch gekennzeichnet, daß die Stufe a in wäßrig-organischer Lösung bei einer Temperatur zwischen 60 und 100°C durchgeführt wird unter Verwendung von Hydroxylaminhydrochlorid oder Hydroxylaminsulfat als Hydroxylaminsalz.

8. Verfahren gemäß Anspruch 6, dadurch gekennzeichnet, daß die Stufe b durch Einwirkung einer starken konzentrierten Säure bei einer Temperatur zwischen 100 und 150°C bewirkt wird.

9. Verfahren gemäß Anspruch 6, dadurch gekennzeichnet, daß die Stufe c durchgeführt wird, indem das Reaktionsgemisch, das von der Stufe b resultiert, mit Wasser verdünnt wird und indem es dann auf eine Temperatur zwischen 100 und 150°C erhitzt wird.

10. Verfahren zum Modifizieren des Wachstums der Pflanzen, dadurch gekennzeichnet, daß man auf diese Pflanzen eine wirksame Menge von mindestens einer Verbindung der Formel

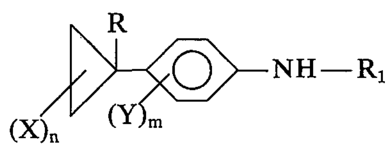

worin X, Y, m, n, R und $R_1$ dieselbe Bedeutung wie in Anspruch 1 haben oder eines Salzes einer dieser Verbindungen aufbringt.

**Patentansprüche für den Vertragsstaat: AT**

1. Zusammensetzung zur Regulierung des Wachstums von Pflanzen, die in der Landwirtschaft verwendbar ist, dadurch gekennzeichnet, daß sie als aktives Material enthält wenigstens:
ein Cyclopropylanilinderivat der Formel

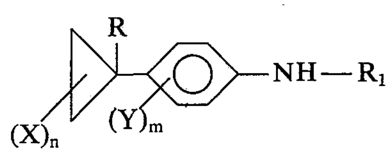

worin:

— R ein Wasserstoffatom oder einen Alkylrest mit 1 bis 4 Kohlenstoffatomen bedeutet,
— X und Y, welche identisch oder verschieden sind, jeweils ein Halogenatom bedeuten,
— $R_1$ ein Wasserstoffatom oder einen Alkylrest mit 1 bis 6 Kohlenstoffatomen bedeutet,
— n eine ganze Zahl gleich Null, 1 oder 2 ist, wobei, falls n größer als 1 ist, die Atome X identisch oder verschieden sein können,
— m eine ganze Zahl gleich Null, 1, 2, 3 oder 4 ist, wobei, falls m größer als 1 ist, die Atome Y identisch oder verschieden sein können,

oder ein Salz dieses Cyclopropylanilinderivats.

2. Zusammensetzung gemäß Anspruch 1, dadurch gekennzeichnet, daß sie als aktives Material enthält wenigstens:

ein Cyclopropylanilinderivat der Formel

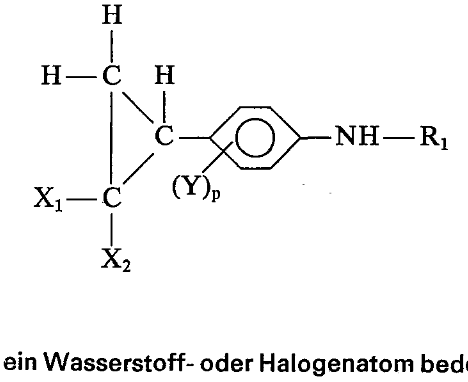

worin:

— $X_1$ ein Wasserstoff- oder Halogenatom bedeutet,

— $X_2$ ein Halogenatom bedeutet,
— Y und $R_1$ dieselbe Bedeutung wie in Anspruch 1 haben,
— und p gleich Null, 1 oder 2 ist,

oder ein Salz dieses Cyclopropylanilinderivats.

3. Zusammensetzung gemäß einem der Ansprüche 1 und 2, dadurch gekennzeichnet, daß sie von 0,001 bis 95 Gew.-% aktives Material sowie einen Träger und/oder oberflächenaktives Mittel enthält.

4. Verfahren zur Herstellung eines Cyclopropylanilinderivats der Formel

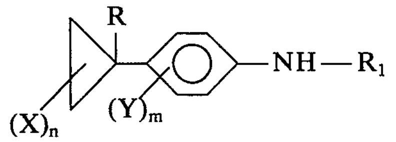

worin R, X, Y, $R_1$, m und n dieselbe Bedeutung wie in Anspruch 1 haben, dadurch gekennzeichnet, daß es die folgenden Stufe a, b, c und gegebenenfalls die Stufe d umfaßt:

a. Einwirkung eines Hydroxylaminsalzes in Gegenwart von Natriumhydroxyd auf das Cyclopropylacetophenon der Formel

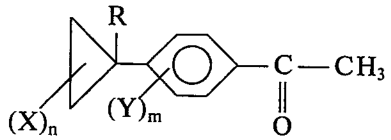

um ein Cyclopropylacetophenoxim der Formel

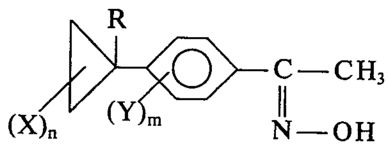

worin X, Y, m, n und R dieselbe Bedeutung wie in Anspruch 1 haben, zu ergeben,

b. Umlagerung des von der Stufe a resultierenden Cyclopropylacetophenoxims, um ein Cyclopropylacetanilid der Formel

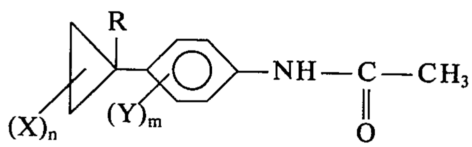

zu ergeben, worin X, Y, m, n und R dieselbe Bedeutung wie in Stufe a haben,

c. Hydrolyse des von der Stufe b resultierenden Cyclopropylacetanilins, um ein Cyclopropylanilin der Formel

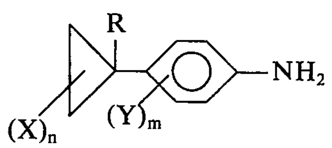

zu ergeben, worin X, Y, m, n und R dieselbe Bedeutung wie in Stufe b haben;

d. Alkylierung der von der Stufe c resultierenden Verbindung, um deren N-Alkylderivat zu ergeben.

5. Verfahren gemäß Anspruch 4, dadurch gekennzeichnet, daß die Stufe a in wäßrig-organischer Lösung bei einer Temperatur zwischen 60 und 100° C durchgeführt wird, wobei als Hydroxylaminsalz das Hydroxylaminhydrochlorid oder das Hydroxylaminsulfat verwendet wird.

6. Verfahren gemäß Anspruch 4, dadurch gekennzeichnet, daß die Stufe b durch Einwirkung einer starken konzentrierten Säure bei einer Temperatur zwischen 100 und 150° C durchgeführt wird.

7. Verfahren gemäß Anspruch 4, dadurch gekennzeichnet, daß die Stufe c durchgeführt wird, indem das von der Stufe b resultierende Reaktionsgemisch mit Wasser verdünnt wird und indem es dann auf

eine Temperatur zwischen 100 und 150° C erhitzt wird.

8. Verfahren zur Herstellung der Salze des Cyclopropylanilinderivats der Formel

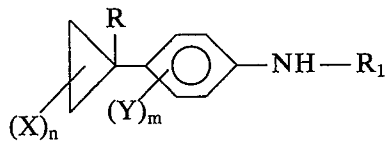

worin R, X, Y, $R_1$ und m dieselbe Bedeutung wie in Anspruch 1 haben, dadurch gekennzeichnet, daß es darin besteht, dieses Derivat mit einer Säure zu behandeln.

9. Verfahren gemäß Anspruch 8, dadurch gekennzeichnet, daß die Säure Salzsäure, Schwefelsäure, Salpetersäure oder phosphorige Säure ist.

10. Verfahren zum Modifizieren des Wachstums von Pflanzen, dadurch gekennzeichnet, daß man auf diese Pflanzen eine wirksame Menge wenigstens einer Verbindung der Formel

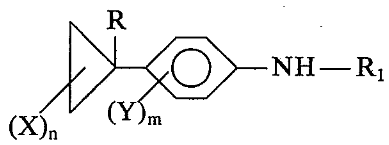

worin X, Y, m, n, R und $R_1$ dieselbe Bedeutung wie in Anspruch 1 haben oder eines Salzes einer dieser Verbindungen, aufbringt.

**Claims for the contracting States: BE, CH, DE, FR, GB, IT, LI, LU, NL**

1. Composition for regulating plant growth, characterised in that it contains, as the active ingredient, at least:

one cyclopropylaniline derivative of the formula:

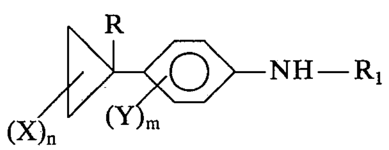

in which:

- R represents a hydrogen atom or an alkyl radical containing from 1 to 4 carbon atoms,
- X and Y, which are identical or different, each represent a halogen atom,
- $R_1$ represents a hydrogen atom or an alkyl radical containing from 1 to 6 carbon atoms,
- n is an integer equal to 0, 1 or 2, it being understood that if n is greater than 1, the atoms X can be identical or different, and
- m is an integer equal to 0, 1, 2, 3 or 4, it being understood that if m is greater than 1, the atoms Y can be identical or different,

or a salt of this cyclopropylaniline derivative.

2. Composition according to Claim 1, characterised in that it contains, as the active ingredient, at least:

one cyclopropylaniline derivative of the formula:

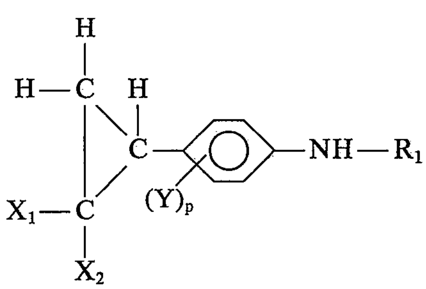

in wich:

- $X_1$ represents a hydrogen or halogen atom,
- $X_2$ represents a halogen atom,
- Y and $R_1$ have the same meaning as in Claim 1, and
- p is equal to 0, 1 or 2,

or a salt of this cyclopropylaniline derivative.

3. Composition according to one of Claims 1 and 2, characterised in that it contains from 0.001 to 95% by weight of active ingredient, together with a carrier and/or surface-active agent.

4. Cyclopropylaniline derivative, characterised in that it corresponds to the formula:

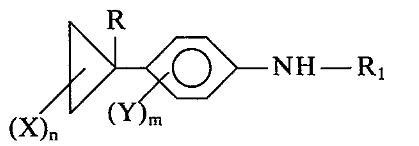

in which R, X, Y, $R_1$, m and n have the same meaning as in Claim 1, and salts of this cyclopropylaniline derivative which are formed by reaction with an acid, with the proviso that this compound is neither 4-(2,2-dichlorocyclopropyl)-aniline nor 4-cyclopropylaniline.

5. Compound according to Claim 4, characterised in that it corresponds to the formula:

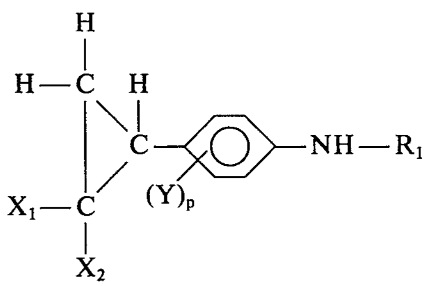

in which $X_1$, $X_2$, Y, $R_1$ and p have the same meaning as in Claim 2, and the salts of this compound.

6. Process for the preparation of a compound according to Claim 4, characterised in that it comprises the following steps a, b and c and, if appropriate, the following step d:

a reaction of a hydroxylamine salt, in the presence of sodium hydroxide, with the cyclopropylacetophenone of the formula:

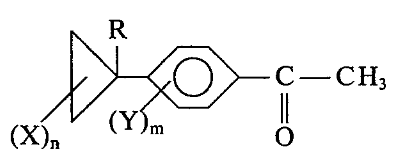

to give a cyclopropylacetophenoxime of the formula:

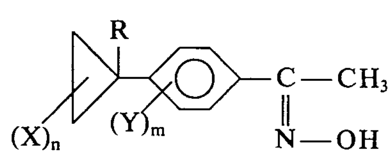

in which formulae X, Y, m, n and R have the same meaning as in Claim 4.

b rearrangement of the cyclopropylacetophenoxime resulting from step a to give a cyclopropylacetanilide of the formula:

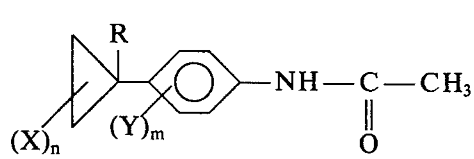

in which X, Y, m, n and R have the same meaning as in step a,

c hydrolysis of the cyclopropylacetanilide resulting from step b to give a cyclopropylaniline of the formula:

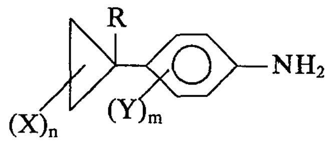

in which X, Y, m, n and R have the same meaning as in step b.

d alkylation of the compound resulting from step c to give its N-alkyl derivative.

7. Process according to Claim 6, characterised in that step a is carried out in aqueous-organic solution, at a temperature of between 60 and 100° C, using hydroxylamine hydrochloride or sulphate as the hydroxylamine salt.

8. Process according to Claim 6, characterised in that step b is carried out by reaction with a highly concentrated acid, at a temperature of between 100 and 150° C.

9. Process according to Claim 6, characterised in that step c is carried out by diluting with water the reaction mixture resulting from step b, and then by heating it to a temperature of between 100 and 150° C.

10. Process for modifying plant growth, characterised in that an effective amount of at least one compound of the formula:

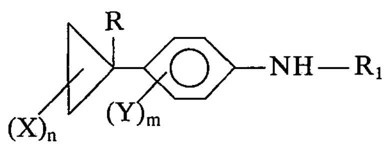

in which X, Y, m, n, R and $R_1$ have the same meaning as in Claim 1, or of a salt of one of these compounds, is applied to these plants.

**Claims for the contracting State: AT**

1. Composition for regulating plant growth, which can be used in agriculture, characterised in that it contains, as the active ingredient, at least:

one cyclopropylaniline derivative of the formula:

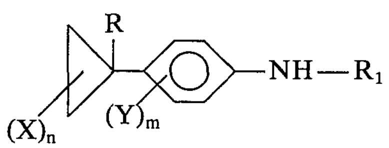

in which:

- R represents a hydrogen atom or an alkyl radical containing from 1 to 4 carbon atoms,
- X and Y, which are identical or different, each represent a halogen atom,
- $R_1$ represents a hydrogen atom or an alkyl radical containing from 1 to 6 carbon atoms,
- n is an integer equal to 0, 1 or 2, it being understood that if n is greater than 1, the atoms X can be identical or different, and
- m is an integer equal to 0, 1, 2, 3 or 4, it being understood that if m is greater than 1, the atoms Y can be identical or different,

or a salt of this cyclopropylaniline derivative.

2. Composition according to Claim 1, characterised in that it contains, as the active ingredient, at least:

one cyclopropylaniline derivative of the formula:

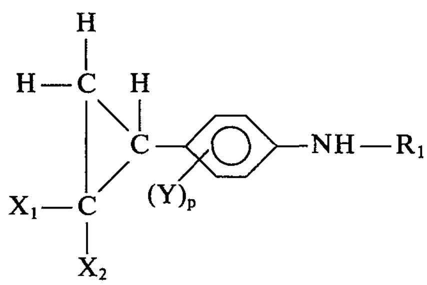

in which:

- $X_1$ represents a hydrogen or halogen atom,
- $X_2$ represents a halogen atom,
- Y and $R_1$ have the same meaning as in Claim 1, and
- p is equal to 0, 1 or 2,

or a salt of this cyclopropylaniline derivative.

3. Composition according to one of Claims 1 and 2, characterised in that it contains from 0.001 to 95% by weight of active ingredient, together with a carrier and/or surface-active agent.

4. Process for the preparation of a cyclopropylaniline derivative corresponding to the formula:

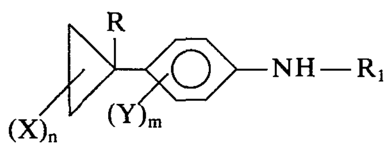

in which R, X, Y, $R_1$, m and n have the same meaning as in Claim 1, characterised in that it comprises the following steps a, b and c and, if appropriate, the following step d:

a reaction of a hydroxylamine salt, in the presence of sodium hydroxide, with the cyclopropylacetophenone of the formula:

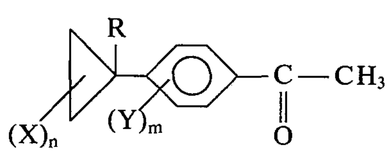

to give a cyclopropylacetophenoxime of the formula:

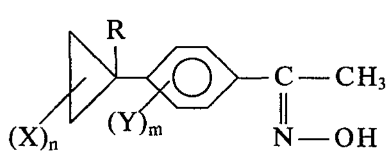

in which formulae X, Y, m, n and R have the same meaning as in Claim 1.

b rearrangement of the cyclopropylacetophenoxime resulting from step a to give a cyclopropylacetanilide of the fotmula:

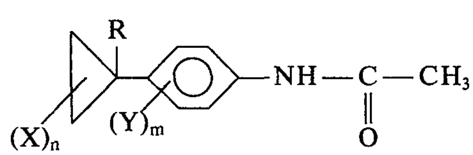

in which X, Y, m, n and R have the same meaning as in step a.

c hydrolysis of the cyclopropylacetanilide resulting from step b to give a cyclopropylaniline of the formula:

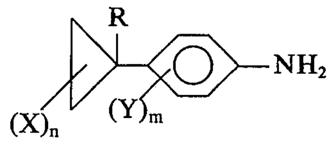

in which X, Y, m, n and R have the same meaning as in step b.

d alkylation of the compound resulting from step c to give its N-alkyl derivative.

5. Process according to Claim 4, characterised in that step a is carried out in aqueous-organic solution, at a temperature of between 60 and 100° C, using hydroxylamine hydrochloride or sulphate as the hydroxylamine salt.

6. Process according to Claim 4, characterised in that step b is carried out by reaction with a highly concentrated acid, at a temperature of between 100 and 150° C.

7. Process according to Claim 4, characterised in that step c is carried out by diluting with water the reaction mixture resulting from step b, and then by heating it to a temperature of between 100 and 150° C.

8. Process for the preparation of the salts of the cyclopropylaniline derivative corresponding to the formula:

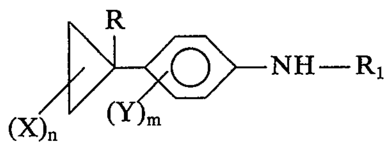

in which R, X, Y, $R_1$ and m have the same meaning as in Claim 1, characterised in that it consists in treating this derivative with an acid.

9. Process according to Claim 8, characterised in that the acid is hydrochloric acid, sulphuric acid, nitric acid or phosphorous acid.

10. Process for modifying plant growth, characterised in that an effective amount of at least one compound of the formula:

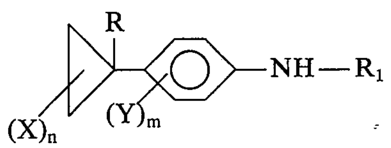

in which X, Y, m, n, R and $R_1$ have the same meaning as in Claim 1, or of a salt of one of these compounds, is applied to these plants.